# NEUE EUROPÄISCHE PATENTSCHRIFT

(11) **EP 0 372 119 B2**
(45) Veröffentlichungstag und Bekanntmachung des Hinweises auf die Entscheidung über den Einspruch: **27.08.1997**
(45) Hinweis auf die Patenterteilung: 02.03.1994
(21) Anmeldenummer: 88120646.0
(22) Anmeldetag: 09.12.1988
(51) Int. Cl.: A61B 17/225

(54) **Lithotripter**
Lithotripter
Lithotripteur

(43) Veröffentlichungstag der Anmeldung: 13.06.1990
(73) Patentinhaber: SIEMENS AKTIENGESELLSCHAFT, 80333 München (DE)
(72) Erfinder: Rattner, Manfred, D-8521 Grossenseebach (DE); Plisek, Franz, D-8520 Erlangen (DE)

(56) Entgegenhaltungen:
- EP-A- 0 273 256
- EP-A- 0 369 177
- EP-A- 0 372 174
- DE-A- 3 736 733
- FR-A- 2 591 467

## Beschreibung

Die Erfindung betrifft einen Lithotripter mit einem Röntgensystem zur Ortung von Konkrementen und einem Stoßwellenkopf zur Erzeugung von Stoßwellen für die Zertrümmerung der Konkremente.

Es ist ein Lithotripter dieser Art bekannt, bei dem zwei Röntgensysteme mit je einem Röntgenstrahler und einem Röntgenbildverstärker mit nachgeschalteter Fernsehkette vorhanden sind (EP-A-0 273 256). Der Patient wird dabei aus zwei Richtungen durchstrahlt, so daß eine Ortung der Konkremente möglich ist. Zwei Stoßwellenköpfe sind derart verstellbar gelagert, daß jeweils einer von ihnen zur Behandlung seitlich an den Patienten herangefahren werden kann. Der Zentralstrahl der ausgesandten Stoßwellen und die Zentralstrahlen der beiden Röntgensysteme schneiden sich dabei in einem Isozentrum, in dem das zu behandelnde Konkrement positioniert wird. Da die Ebene, die durch die beiden Zentralstrahlen der Schallkeulen der Stoßwellenköpfe begrenzt ist, senkrecht zu der durch die beiden Zentralstrahlen der Röntgensysteme begrenzten Ebene liegt, ist der seitliche Platzbedarf relativ groß.

Durch die FR-A-2 591 467 ist ein Lithotripter mit einem Ultraschall-Ortungssystem bekannt, bei dem der Stoßwellengenerator das Ultraschall-Ortungssystem umschließt. Für die Ortung mit Röntgenstrahlung ist dieses Prinzip nicht anwendbar.

In der prioritätsälteren, aber nicht vorveröffentlichten EP 0 369 177 A2 ist ein Lithotripter mit einem Röntgensystem zur Ortung von Konkrementen und einem Stoßwellenkopf zur Erzeugung von Stoßwellen beschrieben, dessen Stoßwellenkopf einen von der Röntgenstrahlung durchsetzten, zentrischen, hohlzylindrischen Raum ohne Strahlenschwächung aufweist. Außerdem ist in dieser Druckschrift ein Lithotripter beschrieben, der statt des Röntgensystems ein Ultraschall-Ortungssystem aufweist, das an der gleichen Stelle angeordnet ist, an der sich im Falle des zuvor erwähnten Ausführungsbeispieles der von der Röntgenstrahlung durchsetzte, zentrische, hohlzylindrische Raum ohne Strahlenschwächung befindet.

In der ebenfalls prioritätsälteren, jedoch nicht vorveröffentlichten EP 0 372 174 A1 ist ein Lithotripter mit einem Röntgensystem und einem Ultraschall-Ortungssystem zur Ortung von Konkrementen sowie einem Stoßwellenkopf zur Erzeugung von Stoßwellen für die Zertrümmerung der Konkremente beschrieben, der einen von der Röntgenstrahlung durchsetzten, zentrischen, hohlzylindrischen Raum ohne Strahlenschwächung aufweist. Das Ultraschall-Ortungssystem ist fest mit dem Stoßwellenkopf verbunden und in einer Bohrung des Stoßwellenkopfes aufgenommen. Dabei ist die Anordnung derart getroffen, daß sich die Achse des Ultraschall-Ortungssystems und der durch den genannten Raum verlaufende Zentralstrahl des Röntgensystems schneiden.

Der Erfindung liegt die Aufgabe zugrunde, einen Lithotripter der eingangs genannten Art so auszubilden, daß sich ein kompakter Aufbau ergibt.

Diese Aufgabe ist erfindungsgemäß gelöst durch die Merkmale der Patentansprüche 1, 5 und 7. Bei dem erfindungsgemäßen Lithotripter kann der Stoßwellenkopf in Strahlenrichtung gesehen nach dem Röntgenstrahler angeordnet werden. Seitlicher Platzbedarf unter dem Patientenlagerungstisch ist nicht erforderlich.

Der Stoßwellenkopf weist einen von der Röntgenstrahlung durchsetzten, zentrischen, hohlzylindrischen Bereich ohne Strahlenschwächung auf, z.B. einen luftgefüllten Raum. Dadurch ist eine besonders gute Bildgebung und die einfache Mitbeobachtung während der Therapie im beschallten Bereich möglich. Der Stoßwellenkopf kann an der Primärstrahlenblende des Röntgensystems oder auch vor dem Strahlenempfänger, z.B. dem Bildverstärker, des Röntgensystems angeordnet werden.

Die Erfindung ist nachfolgend anhand eines in der Zeichnung dargestellten Ausführungsbeispiels näher erläutert. Es zeigen:
- Fig. 1: einen Lithotripter zur Erläuterung des Erfindungsgedankens, und
- Fig. 2: den Stoßwellenkopf des Lithotripters gemäß Fig. 1.

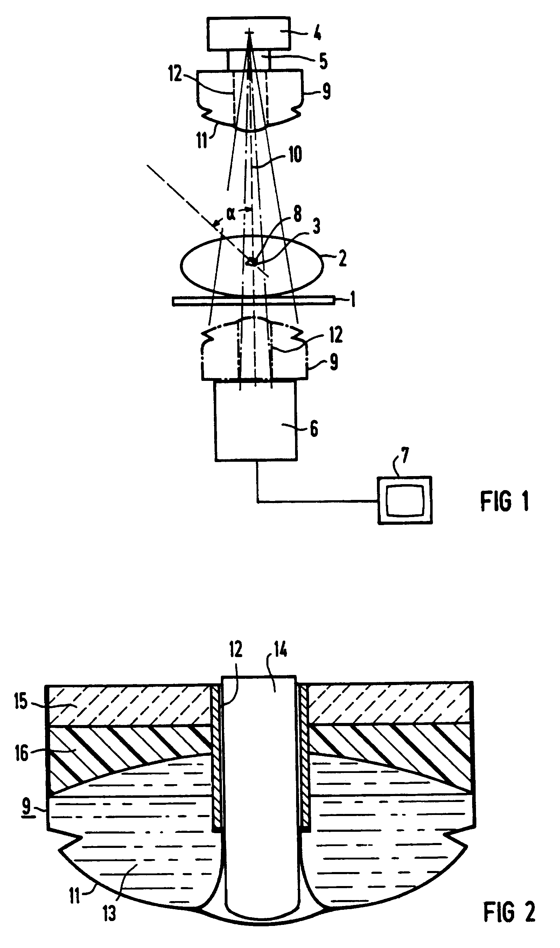

In der Fig. 1 ist eine Liege 1 dargestellt, auf der ein Patient 2 liegt, der ein Konkrement 3 enthält. Das Konkrement 3 soll durch Stoßwellen zertrümmert werden. Zur Ortung des Konkrements 3 ist ein Röntgensystem mit einem Röntgenstrahler 4 mit Primärstrahlenblende 5 und einem Röntgenbildverstärker 6 mit nachgeschalteter Fernsehkette 7 vorgesehen. Damit eine räumliche Information über die Lage des Konkrements 3 erhalten wird, kann das Röntgensystem 4, 5, 6 um eine Achse 8 um einen Winkel α geschwenkt werden, so daß eine Durchstrahlung des Patienten 2 unter verschiedenen Richtungen möglich ist. Die Achse 8 soll dabei mit dem Konkrement 3 zusammenfallen. Hierzu ist die Liege 1 entsprechend verstellbar.

Nach der Ortung und Einstellung des Konkrements 3 in der geschilderten Weise erfolgt die Beschallung des Konkrements 3 mit Stoßwellen. Hierzu ist an der Primärstrahlenblende 5 ein Stoßwellenkopf 9 angebracht, der auf einen Fokusbereich fokusssierte Stoßwellen aussendet und so eingestellt wird, daß der Fokusbereich ebenfalls mit dem Konkrement 3 zusammenfällt. Hierzu kann der Stoßwellenkopf 9 in Richtung des Zentralstrahls 10 des Röntgensystems 4, 5, 6 verstellbar gelagert sein. Die Verstellung kann dabei individuell, aber auch zusammen mit dem Röntgenstrahler 4 erfolgen. Der Stoßwellenkopf 9 ist an seiner Applikationsseite von einer flexiblen Folie 11 abgedeckt, die zur akustischen Kopplung an der Oberfläche des Patienten 2 angelegt wird.

Wesentlich ist, daß der Stoßwellenkopf 9 in Bezug auf das Röntgensystem 4, 5, 6 derart angeordnet ist, daß der Zentralstrahl 10 des Röntgensystems 4, 5, 6 etwa zentrisch durch den Stoßwellenkopf 9 verläuft. Hierzu besitzt der Stoßwellenkopf 9 einen von der Röntgenstrahlung durchsetzten, zentrischen, hohlzylindrischen Raum 12 ohne Strahlenschwächung. Der Raum 12 ist luftgefüllt. Seine hohlzylindrische Wandung begrenzt den mit Koppelflüssigkeit gefüllten Raum des Stoßwellenkopfes 9. Dies ist nachfolgend anhand der Fig. 2 noch näher erläutert.

Bei dem in Fig. 1 dargestellten Lithotripter fällt der Zentralstrahl 10 des Röntgensystems 4, 5, 6 mit dem Zentralstrahl der von dem Stoßwellenkopf 9 ausgesandten Schallkeule zusammen. Die Ortung erfolgt durch Verschwenken des Röntgensystems 4, 5, 6. Es ist auch möglich, ein zweites Röntgensystem vorzusehen, das unter dem Winkel α gegenüber dem Zentralstrahl 10 einstrahlt, und das Röntgensystem 4, 5, 6 ortsfest anzuordnen. Ferner ist es möglich, den Röntgenstrahler 4 zur Anfertigung von Röntgen-Stereo-Bildern auszubilden, indem zwei wechselweise strahlende Foken vorgesehen werden, so daß die beiden Zentralstrahlen, die von diesen beiden Foken ausgehen, einen kleinen Winkel miteinander bilden. In diesem Fall muß der Raum 12 in seinen Abmessungen so ausgebildet werden, daß die von beiden Foken ausgesandten Röntgenstrahlenbündel voll den Patienten 2 durchsetzen.

Die Fig. 1 zeigt strichpunktiert eine zweite Möglichkeit der Anordnung des Stoßwellenkopfes 9 vor dem Röntgenbildverstärker 6. In diesem Fall ist das zur Bilderzeugung nutzbare Röntgenstrahlenbündel ebenfalls durch die Abmessungen des Raumes 12 festgelegt.

Die Fig. 2 zeigt, daß der Stoßwellenkopf 9 einen geschlossenen, von einer Koppelflüssigkeit, z.B. Wasser, ausgefüllten Raum 13 aufweist. Die Applikationsseite des Stoßwellenkopfes 9 ist von der flexiblen Folie 11 abgedeckt, die über den Raum 12 gezogen ist. Die hohlzylindrische Wandung des Raums 12 begrenzt den mit Koppelflüssigkeit gefüllten Raum 13.

Im Raum 12 ist ein Ultraschall-Ortungssystem 14 angeordnet, das in axialer Richtung aus dem Raum 12 herausgezogen werden kann. Der Raum 12 ist - wie oben ausgeführt - luftgefüllt und seine hohlzylindrische Wandung begrenzt den mit Koppelflüssigkeit gefüllten Raum 13, so daß beim Herausziehen des Ultraschall-Ortungssystems 14 keinerlei Maßnahmen gegen das Herauslaufen von Koppelflüssigkeit getroffen werden müssen.

Im Stoßwellenkopf 9 ist eine Stoßwellenquelle 15 vorgesehen, die z.B. piezokeramischer oder elektrodynamischer Art sein kann. Sie sendet fokussierte Stoßwellen aus, die durch ein akustisches Linsensystem 16, das zur Veränderung der Lage des Fokusbereichs einstellbar sein kann, fokussiert wird.

## Patentansprüche

1. Lithotripter mit einem Röntgensystem (4, 5, 6) zur Ortung von Konkrementen (3) und einem Stoßwellenkopf (9) zur Erzeugung von Stoßwellen für die Zertrümmerung der Konkremente (3), dessen Stoßwellenkopf (9) einen von der Röntgenstrahlung durchsetzten, zentrischen, hohlzylindrischen Raum (12) ohne Strahlenschwächung aufweist, in dem ein Ultraschall-Ortungssystem (14) angeordnet ist, das in axialer Richtung aus dem Raum (12) herausgezogen werden kann.

2. Lithotripter nach Anspruch 1, bei welchem der Zentralstrahl (10) des Röntgensystems (4, 5, 6) mit dem Zentralstrahl der von dem Stoßwellenkopf (9) ausgesandten Schallkeule zusammenfallt.

3. Lithotripter nach Anspruch 1, dessen Stoßwellenkopf (9) an der Primärstrahlenblende (5) angeordnet ist.

4. Lithotripter nach Anspruch 1, dessen Stoßwellenkopf (9) vor dem Strahlenempfänger (6) des Röntgensystems (4, 5, 6) angeordnet ist.

5. Lithotripter mit einem Röntgensystem (4, 5, 6) zur Ortung von Konkrementen (3) und einem Stoßwellenkopf (9) zur Erzeugung von Stoßwellen für die Zertrümmerung der Konkremente (3), dessen Stoßwellenkopf (9) einen von der Röntgenstrahlung durchsetzten, zentrischen, hohlzylindrischen Raum (12) ohne Strahlenschwächung aufweist und dessen Stoßwellenkopf (9) an der Primärstrahlenblende (5) angebracht ist.

6. Lithotripter nach Anspruch 5, bei welchem der Zentralstrahl (10) des Röntgensystems (4, 5, 6) mit dem Zentralstrahl der von dem Stoßwellenkopf (9) ausgesandten Schallkeule zusammenfällt.

7. Lithotripter mit einem Röntgensystem (4, 5, 6) zur Ortung von Konkrementen (3) und einem Stoßwellenkopf (9) zur Erzeugung von Stoßwellen für die Zertrümmerung der Konkremente (3), dessen Stoßwellenkopf (9) einen von der Röntgenstrahlung durchsetzten, zentrischen, hohlzylindrischen Raum (12) ohne Strahlenschwächung aufweist, und dessen Stoßwellenkopf (9) derart vor dem Strahlenempfänger (6) des Röntgensystems (4, 5, 6) angeordnet ist, daß sich im Betrieb des Lithotripters der Patient (2) zwischen dem Stoßwellenkopf (9) und dem Röntgenstrahler (4) des Röntgensystems (4, 5, 6) befindet.

8. Lithotripter nach Anspruch 7, bei welchem der Zentralstrahl (10) des Röntgensystems (4, 5, 6) mit dem Zentralstrahl der von dem Stoßwellenkopf (9) ausgesandten Schallkeule zusammenfällt.

## Claims

1. Lithotriptor having an X-ray system (4, 5, 6) for locating calculi (3) and a shock wave head (9) for generating shock waves for disintegrating the calculi (3), the shock wave head (9) having a central, hollow cylindrical chamber (12) through which the X-radiation passes without attenuation and in which there is arranged an ultrasonic location system (14) which can be withdrawn from the chamber (12) in the axial direction.

2. Lithotriptor according to claim 1, in which the central beam (10) of the X-ray system (4, 5, 6) coincides with the central beam of the sound cone emitted by the shock wave head (9).

3. Lithotriptor according to claim 1, whose shock wave head (9) is arranged on the primary radiation diaphragm (5).

4. Lithotriptor according to claim 1, whose shock wave head (9) is arranged in front of the radiation detector (6) of the X-ray system (4, 5, 6).

5. Lithotriptor having an X-ray system (4, 5, 6) for locating calculi (3) and a shock wave head (9) for generating shock waves for disintegrating the calculi (3), the shock wave head (9) having a central, hollow cylindrical chamber (12) through which the X-radiation passes without attenuation and the shock wave head (9) being attached to the primary radiation diaphragm (5).

6. Lithotripter according to claim 5, in which the central beam (10) of the X-ray system (4, 5, 6) coincides with the central beam of the sound cone emitted by the shock wave head (9).

7. Lithotriptor having an X-ray system (4, 5, 6) for locating calculi (3) and a shock wave head (9) for generating shock waves for disintegrating the calculi (3), the shock wave head (9) having a central, hollow cylindrical chamber (12) through which the X-radiation passes without attenuation and the shock wave head (9) being arranged in front of the radiation detector (6) of the X-ray system (4, 5, 6) in such a way that, during operation of the lithotriptor, the patient (2) is located between the shock wave head (9) and the X-ray emitter (4) of the X-ray system (4, 5, 6).

8. Lithotriptor according to claim 7, in which the central beam (10) of the X-ray system (4, 5, 6) coincides with the central beam of the sound cone emitted by the shock wave head (9).

## Revendications

1. Appareil de lithotritie comportant un système radiologique (4,5,6) pour localiser des concrétions (3) et une tête (9) de production d'ondes de choc servant à produire des ondes de choc pour la fragmentation des concrétions (3), la tête (9) de production d'ondes de choc comportant un espace (12) cylindrique creux centré, traversé par le rayonnement X, sans affaiblissement du rayonnement, dans lequel est disposé un système (14) de localisation d'ultrason qui peut être retiré de l'espace (12) suivant une direction axiale.

2. Appareil de lithotritie suivant la revendication 1, caractérisé par le fait que le rayon central (10) du système radiologie (4,5,6) coïncide avec le rayon central du lobe acoustique émis par la tête (9) de production d'ondes de choc.

3. Appareil de lithotritie suivant la revendication 1, caractérisé par le fait que la tête (9) de production d'ondes de choc est disposée sur le diaphragme (5) du rayonnement primaire.

4. Appareil de lithotritie suivant la revendication 1, caractérisé par le fait que la tête (9) de production d'ondes de choc est disposée devant le récepteur de rayonnement (6) du système radiologie (4,5,6).

5. Appareil de lithotritie comportant un système (4,5,6) radiologie pour localiser des concrétions (3) et une tête (9) de production d'ondes de choc servant à produire des ondes pour la fragmentation des concrétions (3), la tête (9) de production d'ondes de choc comportant un espace (12) cylindrique creux centré, traversé par le rayonnement X sans affaiblissement du rayonnement et la tête (5) de production d'ondes de choc étant montée sur le diaphragme (5) du rayonnement primaire.

6. Appareil de lithotritie suivant la revendication 5, caractérisé par le fait que le rayon central (10) du système radiologie (4,5,6) coïncide avec le rayon central du lobe acoustique émis par la tête (9) de production d'ondes de choc.

7. Appareil de lithotritie comportant un système radiologique (4,5,6) pour localiser des concrétions (3) et une tête (9) de production d'ondes de choc servant à produire des ondes de choc pour la fragmentation des concrétions (3), la tête (9) de production d'ondes de choc comportant un espace (12) cylindrique creux centré, traversé par le rayonnement X, sans affaiblissement du rayonnement, la tête (9) de production d'ondes de choc étant disposé avant le récepteur (6) de rayonnement du système (4,5,6) radiologique, de telle sorte que le patient (2), lorsque le dispositif de lithotritie fonctionne, se trouve entre la tête (9) de production d'ondes de choc et l'émetteur (4) de rayons X du système (4,5,6) radiologique.

8. Appareil de lithotrie suivant la revendication 7, caractérisé par le fait que le rayon central (10) du système radiologie (4,5,6) coïncide avec le rayon central du lobe acoustique émis par la tête (9) de production d'ondes de choc.
